(19) Europäisches Patentamt
European Patent Office
Office européen des brevets

(11) EP 4 206 244 A1

(12) **EUROPEAN PATENT APPLICATION**
published in accordance with Art. 153(4) EPC

(43) Date of publication:
**05.07.2023 Bulletin 2023/27**

(21) Application number: **21878027.8**

(22) Date of filing: **07.10.2021**

(51) International Patent Classification (IPC):
***C08F 220/34*** *(2006.01)* ***C08F 220/04*** *(2006.01)*
***C08J 3/24*** *(2006.01)* ***A61L 15/60*** *(2006.01)*

(52) Cooperative Patent Classification (CPC):
**A61L 15/60; C08F 220/04; C08F 220/34; C08J 3/24**

(86) International application number:
**PCT/KR2021/013807**

(87) International publication number:
**WO 2022/075777 (14.04.2022 Gazette 2022/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.10.2020 KR 20200129643**

(71) Applicant: **Lg Chem, Ltd.**
**Seoul 07336 (KR)**

(72) Inventors:
- **LEE, Ji Seok**
  **Daejeon 34122 (KR)**
- **CHOI, Hyungsam**
  **Daejeon 34122 (KR)**
- **JUNG, Seonjung**
  **Daejeon 34122 (KR)**
- **YUN, Haesung**
  **Daejeon 34122 (KR)**
- **KIM, Byungguk**
  **Daejeon 34122 (KR)**
- **KIM, Sanggon**
  **Daejeon 34122 (KR)**
- **KANG, Soonhee**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

# (54) SUPER ABSORBENT POLYMER AND PREPARATION METHOD THEREFOR

(57) Provided are a superabsorbent polymer and a preparation method thereof, more particularly, a superabsorbent polymer exhibiting improved bacterial growth-inhibiting property without deterioration in its water retention capacity, and a preparation method thereof.

[FIG. 1]

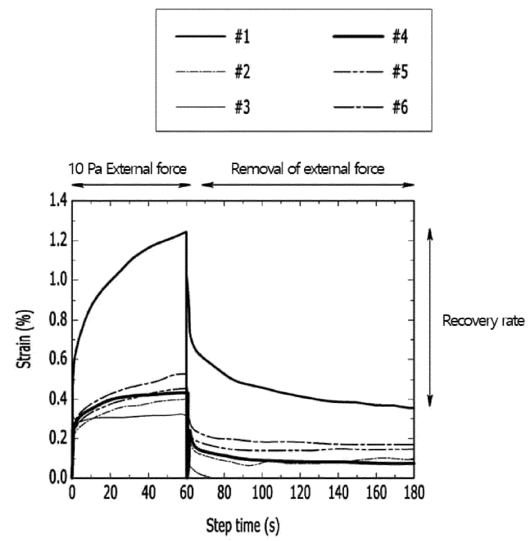


EP 4 206 244 A1

## Description

### [Technical Field]

Cross-reference to Related Application

The present application is based on, and claims priority from, Korean Patent Application Nos. 10-2020-0129643 and 10-2021-0132988, filed on October 7, 2020, and October 7, 2021, respectively, the disclosures of which are hereby incorporated by reference herein in their entirety.

The present invention relates to a superabsorbent polymer exhibiting improved bacterial growth-inhibiting property without deterioration in its water retention capacity, and a preparation method thereof.

### [Background Art]

A superabsorbent polymer (SAP) is a synthetic polymeric material capable of absorbing moisture from 500 to 1000 times its own weight. Various manufacturers have denominated it as different names, such as SAM (Super Absorbency Material), AGM (Absorbent Gel Material), etc. Since such superabsorbent polymers started to be practically applied in sanitary products, now they have been widely used not only for hygiene products such as paper diapers for children, etc., but also for water retaining soil products for gardening, water stop materials for the civil engineering and construction, sheets for raising seedling, fresh-keeping agents for food distribution fields, materials for poultice or the like.

In particular, superabsorbent polymers are most widely applied to hygiene products such as paper diapers for children, diapers for adults, or disposable absorbent products. Therefore, when bacteria grow in these hygiene products and disposable absorbent products, there is a problem in that various diseases are induced as well as secondary odors. Accordingly, attempts have been made to introduce various bacterial growth inhibitory components, or deodorizing or antimicrobial functional components to superabsorbent polymers.

However, in the attempts to introduce antimicrobial agents capable of inhibiting bacterial growth to superabsorbent polymers, it is not easy to select and introduce an antimicrobial agent component that exhibits excellent bacterial growth-inhibiting and deodorizing properties, is harmless to the human body, and satisfies economic feasibility without deteriorating basic physical properties of the superabsorbent polymers.

Accordingly, there is a continued demand for the development of a superabsorbent polymer-related technology capable of inhibiting bacterial growth without deteriorating basic physical properties of superabsorbent polymers.

### [Disclosure]

### [Technical Problem]

Accordingly, there are provided a superabsorbent polymer capable of exhibiting improved bacterial growth-inhibiting property without deterioration in its water retention capacity, and a preparation method thereof.

### [Technical Solution]

According to one embodiment of the present invention, provided is a superabsorbent polymer including a crosslinked polymer of an acrylic acid-based monomer including acidic groups, of which at least a part is neutralized, a polymeric antimicrobial monomer represented by the following Chemical Formula 1, and an internal crosslinking agent:

[Chemical Formula 1]

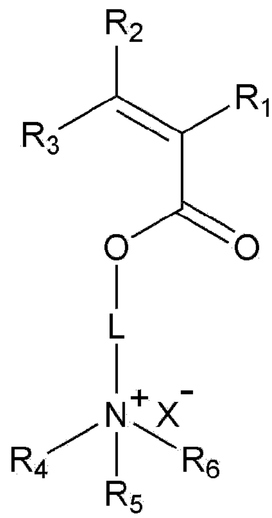

in Chemical Formula 1,

L is alkylene having 1 to 10 carbon atoms,
$R_1$ to $R_3$ are each independently hydrogen or methyl,
among $R_4$ to $R_6$, one is alkyl having 6 to 20 carbon atoms, and the others are each independently alkyl having 1 to 4 carbon atoms, and
X is halogen.

According to another embodiment of the present invention, provided is a method of preparing a superabsorbent polymer, the method including the steps of:

forming a water-containing gel polymer by performing a crosslinking polymerization of an acrylic acid-based monomer including acidic groups, of which at least a part is neutralized, and a polymeric antimicrobial monomer represented by Chemical Formula 1 in the presence of an internal crosslinking agent and a polymerization initiator; and
forming a superabsorbent polymer including a crosslinked polymer by drying, pulverizing, and size-sorting the water-containing gel polymer.

Furthermore, according to still another embodiment of the present invention, provided is a hygiene product including the above-described superabsorbent polymer.

**[Effect of the Invention]**

A superabsorbent polymer of the present invention may exhibit an antimicrobial property of inhibiting bacterial growth which may be harmful to the human body and may cause secondary odors.
Specifically, since the superabsorbent polymer is prepared by using a polymeric antimicrobial monomer having a specific structure during formation of a crosslinked polymer, it may exhibit an antimicrobial property against at least one of Gram-positive bacteria and Gram-negative bacteria while maintaining excellent water retention capacity, unlike those prepared by using another antimicrobial agent, and the used antimicrobial monomer does not remain in the polymer, which does not cause a safety problem in the human body due to leakage of the antimicrobial agent.
Accordingly, the superabsorbent polymer may be very preferably applied to various hygiene products, such as diapers for children as well as diapers for adults required to have an antimicrobial property against bacteria, etc.

**[Brief Description of Drawings]**

FIG. 1 shows a result of a creep test of Experimental Example 2 of the present invention; and
FIGS. 2 and 3 show results of a frequency sweep test and an amplitude sweep test of Example 3 of the present invention, respectively.

**[Best Mode for Carrying Out the Invention]**

The terms used in this description are just for explaining exemplary embodiments and it is not intended to restrict the present invention. The singular expression may include the plural expression unless it is differently expressed contextually. It must be understood that the term "include", "equip", or "have" in the present description is only used for designating the existence of characteristics taken effect, steps, components, or combinations thereof, and do not exclude the existence or the possibility of addition of one or more different characteristics, steps, components or combinations thereof beforehand.

Further, in the present invention, when a layer or an element is mentioned to be formed "on" or "above" layers or elements, it means that each layer or element is directly formed on the layers or elements, or other layers or elements may be formed between the layers, subjects, or substrates.

The present invention may be variously modified and have various forms, and specific examples are illustrated and explained in detail below. However, it is not intended to limit the present invention to the specific examples and it must be understood that the present invention includes every modifications, equivalents, or replacements included in the spirit and technical scope of the present invention.

Further, the technical terminology used herein is for the purpose of describing particular embodiments only and is not intended to be limiting of the invention. Further, the singular forms, as used herein, are intended to include plural forms as well, unless the context clearly indicates otherwise.

Meanwhile, as used herein, the term "(meth)acrylate" includes both acrylates and methacrylates.

Further, as used herein, the alkyl group may be linear or branched, and its number of carbon atoms is, but is not particularly limited to, preferably 1 to 20. According to one embodiment, the number of carbon atoms of the alkyl group is 1 to 10. According to another embodiment, the number of carbon atoms of the alkyl group is 1 to 6. Specific examples of the alkyl group may include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethylpropyl, 1,1-dimethyl-propyl, isohexyl, 2-methylpentyl, 4-methylhexyl, 5-methylhexyl, etc., but are not limited thereto. In the present specification, the description of the above-described alkyl group may be applied to alkylene, except that alkylene is a divalent group.

As used herein, the term "polymer" refers to a polymerized state of acrylic acid-based monomers, and may encompass those of all water content ranges or particle size ranges. Among the polymers, those having a water content (a moisture content) of about 40% by weight or more after being polymerized and before being dried may be designated as a water-containing gel polymer, and particles obtained by pulverizing and drying the water-containing gel polymer may be designated as a crosslinked polymer.

Further, the term "superabsorbent polymer particle" refers to a material in the state of particle, including a crosslinked polymer which is obtained by polymerizing and crosslinking the acrylic acid-based monomer including acidic groups, of which at least a part is neutralized, by an internal crosslinking agent.

Further, the term "superabsorbent polymer" refers to, depending on the context, a crosslinked polymer obtained by polymerizing the acrylic acid-based monomer including acidic groups, of which at least a part is neutralized, or a base polymer in the form of powder, consisting of superabsorbent polymer particles obtained by pulverizing the crosslinked polymer, or is used to encompass those made suitable for commercialization by an additional process of the crosslinked polymer or the base polymer, for example, surface crosslinking, reassembling of fine particles, drying, pulverizing, size-sorting, etc.

In order to secure antimicrobial and deodorizing properties in the traditional superabsorbent polymers, a metal compound having an antimicrobial function or an organic compound containing a cation or alcohol functional group was introduced in the form of an additive. In this case, however, safety of the superabsorbent polymer is lowered, basic physical properties, such as absorption properties, etc., are lowered, and there are problems in persistence of antimicrobial properties and leakage of antimicrobial substances.

For example, an attempt has been made to introduce, to superabsorbent polymers, an antimicrobial agent component containing an antimicrobial metal ion such as silver, copper, copper, zinc, etc., such as copper oxide, etc. This antimicrobial metal ion-containing component may impart deodorizing properties by destroying the cell walls of microorganisms, such as bacteria, etc., and killing bacteria with enzymes that may cause bad odor in the superabsorbent polymers. However, the metal ion-containing component is classified as a biocide material that may kill even microorganisms beneficial to the human body. Thus, when the superabsorbent polymers are applied to hygiene products such as diapers for children or adults, etc., introduction of the metal ion-containing antimicrobial agent component is excluded as much as possible.

Traditionally, when the antimicrobial agent inhibiting bacterial growth was introduced to a superabsorbent polymer, a method of mixing a small amount of the antimicrobial agent with the superabsorbent polymer was mainly

applied. However, when this mixing method is applied, it was difficult to uniformly maintain the bacterial growth-inhibiting property over time. Moreover, this mixing method may cause non-uniform coating property and detachment of the antimicrobial agent component during the process of mixing the superabsorbent polymer and the antimicrobial agent, and there have been also disadvantages such as a need to install a new facility for the mixing.

Further, there are various types of bacteria such that more than 5,000 types of bacteria have been identified. Specifically, bacteria have a variety of cell morphologies such as a sphere shape, a rod shape, a spiral shape, etc., and the oxygen demand is also different between bacteria, and thus bacteria are divided into aerobic bacteria, facultative aerobic bacteria, and anaerobic bacteria. Therefore, it has not been easy for one type of antimicrobial agent to have a physical/chemical mechanism by which cell membranes/cell walls of many different bacteria are damaged or proteins thereof are denatured.

However, it was found that when a superabsorbent polymer is prepared by polymerizing a monomer having a specific structure and containing a quaternary ammonium salt with an acrylic acid-based monomer, it exhibits a water retention capacity above a predetermined level while exhibiting an antimicrobial property against at least one of Gram-positive bacteria and Gram-negative bacteria, more specifically, both Gram-positive bacteria and Gram-negative bacteria, thereby completing the present invention.

Here, "exhibiting an antimicrobial property against particular bacteria" means that the number of bacteria incubated after absorbing the test bacteria-inoculated artificial urine into a superabsorbent polymer, of which antimicrobial property is to be tested, is remarkably decreased, as compared with the number of reference bacteria incubated after absorbing the test bacteria-inoculated artificial urine into a superabsorbent polymer without the antimicrobial material, and specifically, means that an antibacterial rate (%) is 50% or more, as calculated by the following Mathematical Equation 1 according to an antimicrobial property test described later.

[Mathematical Equation 1]

$$\text{Antibacterial rate (\%)} = \left(1 - \frac{C_{sample}}{C_{Refernce}}\right) \times 100$$

wherein $C_{sample}$ represents CFU of bacteria after incubation in a superabsorbent polymer with an antimicrobial material, and $C_{Reference}$ represents CFU of bacteria after incubation in a superabsorbent polymer without the antimicrobial material.

More preferably, "exhibiting an antimicrobial property against particular bacteria" means that the antibacterial rate (%) calculated by Mathematical Equation 1 is 60% or more, 70% or more, 80% or more, 90% or more, 95% or more, or 99% or more.

The Gram-positive bacteria collectively refer to bacteria that are stained violet when stained by the Gram staining method. The cell walls of the Gram-positive bacteria consist of several layers of peptidoglycan, and when stained with a basic dye such as crystal violet, the violet color is not decolorized even though treated with ethanol. Bacteria classified as the Gram-positive bacteria include *Enterococcus faecalis, Staphylococcus aureus, Streptococcus pneumoniae, Enterococcus faecium, Lactobacillus lactis,* etc.

In addition, the Gram-negative bacteria collectively refer to bacteria that are stained red when stained by the Gram staining method. The Gram-negative bacteria have outer membrane consisting of lipopolysaccharides, lipoproteins, and other complex polymeric materials, instead of the cell wall having a relatively small amount of peptidoglycan, as compared with the Gram-positive bacteria. Therefore, when the Gram-negative bacteria are stained with a basic dye such as crystal violet and then treated with ethanol, they are decolorized, and when counter-stained with a red dye such as safranin, they are colored red. Bacteria classified as the Gram-negative bacteria include *Proteus Mirabilis, Escherichia coli, Salmonella typhi, Pseudomonas aeruginosa,* or *Vibrio cholerae,* etc.

Since the Gram-positive bacteria and Gram-negative bacteria may cause various diseases upon contact, and may also cause secondary infection in severe patients with weakened immune systems, it is preferable that a single antimicrobial agent is used to achieve antimicrobial properties against both the Gram-positive bacteria and the Gram-negative bacteria.

Meanwhile, the superabsorbent polymer according to one embodiment includes a repeating unit derived from the antimicrobial monomer represented by Chemical Formula 1 in the main chain constituting the crosslinked polymer, thereby exhibiting antimicrobial property against at least one of Gram-positive bacteria and Gram-negative bacteria. Specifically, due to a quaternary ammonium salt moiety having an alkyl group having a predetermined number or more of carbon atoms in the crosslinked polymer included in the superabsorbent polymer, the ammonium cation of the quaternary ammonium salt is electrostatically adsorbed onto the cell walls of Gram-positive bacteria or Gram-negative bacteria, and then the cell surface structure of bacteria is physically and chemically destroyed by the interaction with

the hydrophobic alkyl group of the quaternary ammonium salt. Thus, the superabsorbent polymer may exhibit the antimicrobial property.

More specifically, the superabsorbent polymer may exhibit the antimicrobial property against one or more types of bacteria classified as the Gram-positive bacteria. Alternatively. the superabsorbent polymer may exhibit the antimicrobial property against one or more types of bacteria classified as the Gram-negative bacteria. Alternatively. the superabsorbent polymer may exhibit the antimicrobial property against one or more types of bacteria classified as the Gram-negative bacteria and one or more types of bacteria classified as the Gram-positive bacteria.

Further, the superabsorbent polymer may exhibit a centrifuge retention capacity(CRC) of 29 g/g to 50 g/g while exhibiting the excellent antimicrobial property as described above. In this regard, when the centrifuge retention capacity of the superabsorbent polymer is less than 29 g/g, the ability to retain a liquid after absorption is reduced, and thus the superabsorbent polymer is not suitable for application to hygiene products. When the centrifuge retention capacity of the superabsorbent polymer is more than 50 g/g, it is not suitable because absorbency under pressure may be lowered, which has a trade-off relationship with the centrifuge retention capacity.

Moreover, in the superabsorbent polymer, the antimicrobial agent forms the main chain of the crosslinked polymer, together with the acrylic acid-based monomer, not in a simple mixed form, and therefore, it does not remain in the form of the antimicrobial monomer compound in the superabsorbent polymer. Accordingly, there is no concern about leakage of the antimicrobial agent even over time.

Hereinafter, a superabsorbent polymer and a preparation method thereof will be described in more detail according to specific embodiments of the present invention.

## Superabsorbent polymer

Specifically, a superabsorbent polymer according to one embodiment of the present invention is characterized by including a crosslinked polymer of an acrylic acid-based monomer including acidic groups, of which at least a part is neutralized; a polymeric antimicrobial monomer; and an internal crosslinking agent, wherein the polymeric antimicrobial monomer includes a compound represented by the following Chemical Formula 1:

[Chemical Formula 1]

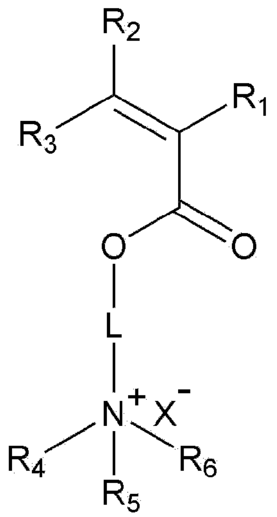

in Chemical Formula 1,

L is alkylene having 1 to 10 carbon atoms,
$R_1$ to $R_3$ are each independently hydrogen or methyl,
among $R_4$ to $R_6$, one is alkyl having 6 to 20 carbon atoms, and the others are each independently alkyl having 1 to 4 carbon atoms, and
X is halogen.

In this regard, the crosslinked polymer, resulting from crosslinking polymerization of the acrylic acid-based monomer including acidic groups, of which at least a part is neutralized, and the polymeric antimicrobial monomer in the presence of an internal crosslinking agent, has a three-dimensional network structure, in which the main chains

formed by polymerization of the monomers are crosslinked by the internal crosslinking agent. Therefore, the polymeric antimicrobial monomer does not exist as a separate compound in the superabsorbent polymer, but exists as a repeating unit constituting the main chain, and thus it does not leak over time. Accordingly, the antimicrobial property of the superabsorbent polymer may be continuously maintained.

Meanwhile, the acrylic acid-based monomer is a compound represented by the following Chemical Formula 1:

[Chemical Formula 2] R-COOM'

in Chemical Formula 2,

R is an alkyl group containing an unsaturated bond and having 2 to 5 carbon atoms, and
M' is a hydrogen atom, a monovalent or divalent metal, an ammonium group, or an organic amine salt.

Preferably, the monomer may include one or more selected from the group consisting of (meth)acrylic acid, and a monovalent (alkaline) metal salt thereof, a divalent metal salt thereof, an ammonium salt thereof, and an organic amine salt thereof.

As described, when (meth)acrylic acid and/or a salt thereof may be used as the acrylic acid-based monomer, it is advantageous in that a superabsorbent polymer having improved absorbency may be obtained.

Here, the acrylic acid-based monomer may have acidic groups, of which at least a part is neutralized. Preferably, those partially neutralized with an alkali substance such as sodium hydroxide, potassium hydroxide, ammonium hydroxide, etc. may be used as the monomer. In this regard, a degree of neutralization of the acrylic acid-based monomer may be 40 mol% to 95 mol%, or 40 mol% to 80 mol%, or 45 mol% to 75 mol%. The range of the neutralization degree may vary depending on final physical properties. However, an excessively high degree of neutralization renders the neutralized monomers precipitated, and thus polymerization may not occur readily, whereas an excessively low degree of neutralization not only greatly deteriorates absorbency of the polymer but also endows the polymer with hard-to-handle properties, such as of elastic rubber.

Meanwhile, the polymeric antimicrobial monomer represented by Chemical Formula 1 includes a linker (L) linked with an acrylic group, which may be polymerized with the acrylic acid monomer, and a quaternary ammonium cation having three terminal groups of $R_4$, $R_5$, and $R_6$ substituents.

In this regard, the linker (L) may be linear alkylene having 1 to 10 carbon atoms. More specifically, L may be linear alkylene having 1 to 5 carbon atoms, for example, methylene, ethylene, or propylene.

Further, one of $R_4$, $R_5$, and $R_6$ substituents which are three terminal groups substituted to the quaternary ammonium cation of the polymeric antimicrobial monomer is alkyl having 6 to 20 carbon atoms. More specifically, one of $R_4$, $R_5$, and $R_6$ substituents is linear, i.e., straight-chain alkyl having 6 to 20 carbon atoms. In this regard, when two of $R_4$, $R_5$, and $R_6$ substituents are alkyl having 1 to 4 carbon atoms, and the other one is alkyl having 5 or less carbon atoms, there is a problem in that the antimicrobial property may not be achieved. When one of $R_4$, $R_5$, and $R_6$ substituents is alkyl having more than 20 carbon atoms, the starting material for preparing the monomer is not dissolved in a solvent, and thus synthesis itself is impossible.

Further, in Chemical Formula 1, one of $R_4$ to $R_6$ is alkyl having 5 to 20 carbon atoms, and the others are each independently methyl or ethyl.

More specifically, $R_1$ is hydrogen, or methyl, $R_2$ and $R_3$ are hydrogen, one of $R_4$ to $R_6$ is alkyl having 5 to 20 carbon atoms, and the others are each independently methyl or ethyl; or
$R_1$ to $R_3$ are all hydrogens, one of $R_4$ to $R_6$ is alkyl having 5 to 20 carbon atoms and the others are each independently methyl or ethyl.

Further, among $R_4$, $R_5$, and $R_6$ substituents, the two substituents other than alkyl having 5 to 20 carbon atoms may be the same as each other.

Preferably, in Chemical Formula 1, one of $R_4$, $R_5$ and $R_6$ may have 6 or more, 7 or more, or 8 or more carbon atoms, and 20 or less, 18 or less, 16 or less, 14 or less, or 12 or less carbon atoms. The antimicrobial copolymer including the first repeating unit represented by Chemical Formula 1 may exhibit more excellent antimicrobial property.

For example, $R_1$ may be methyl, $R_2$ and $R_3$ may be hydrogens, and one of $R_4$, $R_5$ and $R_6$ may be alkyl having 6 to 16 carbon atoms, and the others may be each independently methyl or ethyl. The antimicrobial copolymer including the first repeating unit having such a structure may exhibit excellent antimicrobial property against at least one of Gram-positive bacteria and Gram-positive bacteria, more specifically, all of Gram-positive bacteria and Gram-negative bacteria.

Further, for example, $R_1$ may be methyl, $R_2$ and $R_3$ may be hydrogens, and one of $R_4$ to $R_6$ may be alkyl having 10 to 14 carbon atoms, and the others may be methyl. When the superabsorbent polymer according to one embodiment includes the crosslinked polymer by the polymeric antimicrobial monomer having such a structure, it may exhibit excellent antimicrobial property against at least one of Gram-positive bacteria and Gram-positive bacteria, more specifically, all of Gram-positive bacteria and Gram-negative bacteria even though the polymeric antimicrobial monomer in the

crosslinked polymer is included in a small amount such as 0.1 part by weight or more and 5 parts by weight or less with respect to 100 parts by weight of the acrylic acid-based monomer.

Further, in Chemical Formula 1, X may be halogen, preferably, chloro (Cl) or bromo (Br).

Further, the polymeric antimicrobial monomer may be a compound represented by any one of the following Chemical Formulae 1-1 to 1-4:

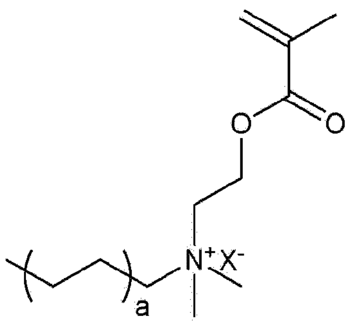

**1-1**

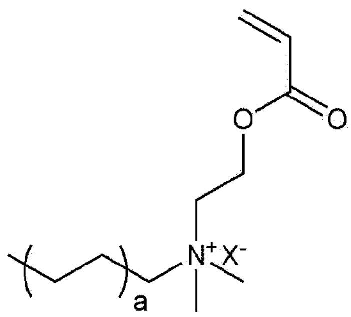

**1-2**

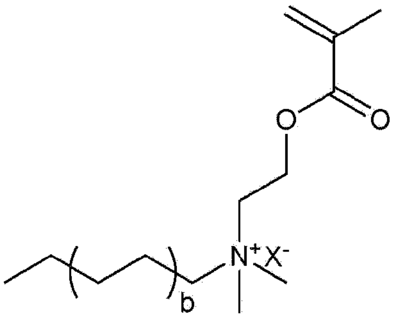

**1-3**

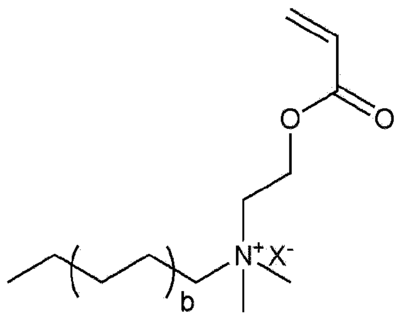

**1-4**

in Chemical Formulae 1-1 to 1-4,

a is an integer of 2 to 9,
b is an integer of 2 to 8, and
X is bromo or chloro.

More specifically, in Chemical Formulae 1-1 to 1-4, a may be 2, 3, 4, 5, 6, 7, 8, or 9, and b may be 2, 3, 4, 5, 6, 7, or 8. Preferably, a and b may be each independently 4, 5, or 6.

For example, the polymeric antimicrobial monomer may be any one selected from the group consisting of the following compounds:

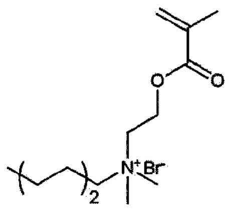
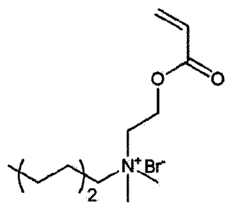
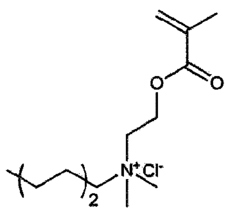
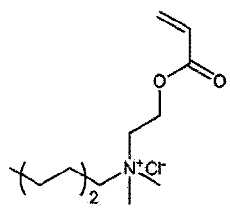

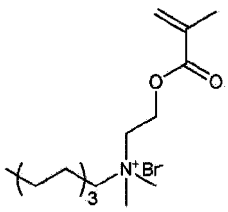 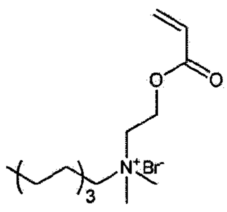 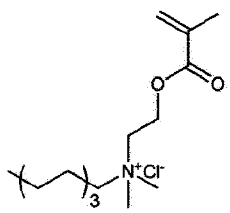 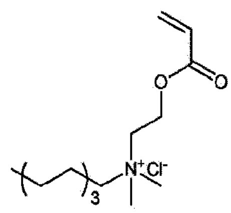

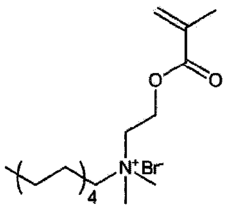 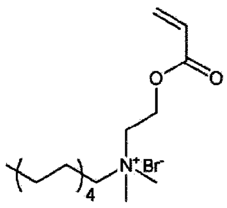 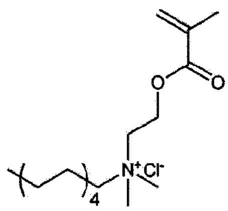 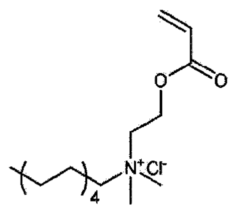

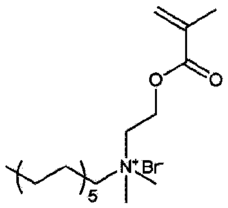 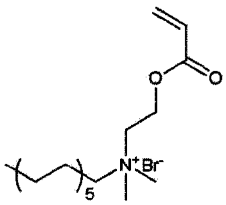 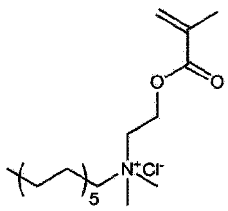 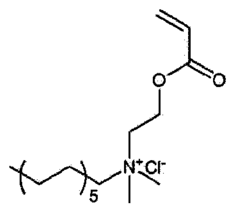.

In the crosslinked polymer, such a polymeric antimicrobial monomer is included in an amount of 0.1 part by weight to 20 parts by weight with respect to 100 parts by weight of the acrylic acid-based monomer. When the polymeric antimicrobial monomer is included in an amount of less than 0.1 part by weight with respect to 100 parts by weight of the acrylic acid-based monomer, it is difficult to achieve sufficient antimicrobial and deodorizing properties. When the polymeric antimicrobial monomer is included in an amount of more than 20 parts by weight with respect to 100 parts by weight of the acrylic acid-based monomer, it may damage a user's normal cells as well as the microorganisms, and thus it is not suitable in terms of human safety, and there is a problem of deteriorating the water retention capacity, which is the general physical property of the superabsorbent polymer. For example, in the crosslinked polymer, the polymeric antimicrobial monomer may be included in an amount of 0.1 part by weight or more, 0.2 parts by weight or more, 0.3 parts by weight or more, and 20 parts by weight or less, 15 parts by weight or less, 10 parts by weight or less, or 5 parts by weight or less with respect to 100 parts by weight of the acrylic acid-based monomer.

In this regard, in the crosslinked polymer, the polymeric antimicrobial monomer may be included in an amount of 0.1 part by weight to 20 parts by weight with respect to 100 parts by weight of the acrylic acid-based monomer, which means that during preparation of the crosslinked polymer, the polymeric antimicrobial monomer is used in an amount of 0.1 part by weight to 20 parts by weight with respect to 100 parts by weight of the acrylic acid-based monomer. In other words, when the residual monomer of the superabsorbent polymer was examined, no leakage of the antimicrobial monomer was observed, indicating that the entire amount of the antimicrobial monomer was used in the polymerization with the acrylic acid-based monomer, which may be confirmed in the Experimental Example to be described later.

For example, the superabsorbent polymer including the polymeric antimicrobial monomer, in which in Chemical Formula 1, one of $R_4$ to $R_6$ is alkyl having 10 to 20 carbon atoms, and the others are methyl, in an amount of 0.1 part by weight to 1.0 part by weight with respect to 100 parts by weight of the acrylic acid-based monomer in the crosslinked polymer may exhibit a 30 min-centrifuge retention capacity (CRC) of 40 g/g to 50 g/g for physiological saline (0.9 wt% aqueous sodium chloride solution), as measured in accordance with EDANA standard WSP 241.3 while exhibiting excellent antimicrobial property against at least one of Gram-positive bacteria and Gram-positive bacteria, preferably, all of them, i.e., an antibacterial rate of 99% or more, as calculated by Mathematical Equation 1.

Further, as used herein, the term 'internal crosslinking agent' is used to distinguish it from a surface crosslinking agent which crosslinks the surface of the superabsorbent polymer particle, described later, and functions to polymerize the acrylic acid-based monomers by crosslinking unsaturated bonds thereof. The crosslinking in the above step is performed regardless of surface or internal crosslinking. However, when the surface crosslinking process of the superabsorbent polymer particles to be described later is performed, the particle surface of the final prepared superabsorbent polymer has a crosslinked structure by the surface crosslinking agent, and the inside thereof has a crosslinked structure by the internal crosslinking agent.

As the internal crosslinking agent, any compound is possible as long as it enables introduction of crosslinkage during polymerization of the acrylic acid-based monomer. Non-limiting examples of the internal crosslinking agent may

include multifunctional crosslinking agents, such as N,N'-methylenebisacrylamide, trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin, or ethylene carbonate, which may be used alone or in combination of two or more thereof, but are not limited thereto. Among them, ethylene glycol diglycidyl ether may be preferably used.

The crosslinking polymerization of the acrylic acid-based monomer in the presence of the internal crosslinking agent may be performed by thermal polymerization, photopolymerization, or hybrid polymerization in the presence of a polymerization initiator, if necessary, a thickener, a plasticizer, a preservation stabilizer, an antioxidant, etc., and a detailed description thereof will be described later.

Further, the superabsorbent polymer may be in the form of particles having a particle size of 850 μm or less, for example, about μm 150 to 850 μm. In this regard, the particle size may be measured in accordance with European Disposables and Nonwovens Association (EDANA) standard WSP 220.3. Here, when the superabsorbent polymer includes a large amount of fine particles having a particle size of less than 150 μm, general physical properties of the superabsorbent polymer may be deteriorated, which is not preferred.

Meanwhile, the superabsorbent polymer may further include a surface-crosslinked layer formed on the crosslinked polymer by additionally crosslinking the crosslinked polymer via a surface crosslinking agent. This is to increase the surface crosslinking density of the superabsorbent polymer particles. As described, when the superabsorbent polymer particle further includes the surface-crosslinked layer, it may have a structure in which the external crosslinking density is higher than the internal crosslinking density.

As the surface crosslinking agent, surface crosslinking agents which have been used in the preparation of the superabsorbent polymer may be used without particular limitation. For example, the surface crosslinking agent may include one or more polyols selected from the group consisting of ethylene glycol, propylene glycol, 1,3-propanediol, 1,4-butanediol, 1,6-hexanediol, 1,2-hexanediol, 1,3-hexanediol, 2-methyl-1,3-propanediol, 2,5-hexanediol, 2-methyl-1,3-pentanediol, 2-methyl-2,4-pentanediol, tripropylene glycol, and glycerol; one or more carbonate-based compounds selected from the group consisting of ethylene carbonate, propylene carbonate, and glycerol carbonate; epoxy compounds such as ethylene glycol diglycidyl ether, etc.; oxazoline compounds such as oxazolidinone, etc.; polyamine compounds; oxazoline compounds; mono-, di-, or polyoxazolidinone compounds; or cyclic urea compounds, etc.

Specifically, as the surface crosslinking agent, one or more, two or more, or three or more of the above-described surface crosslinking agents may be used, and for example, ethylene carbonate-propylene carbonate (ECPC), propylene glycol, and/or glycerol carbonate may be used.

Further, the above-described superabsorbent polymer may have a centrifuge retention capacity (CRC) in the range of 29 g/g or more, 33 g/g or more, 38 g/g or more, or 40 g/g or more, and 50 g/g or less, or 48 g/g or less, 46 g/g or less, or 44 g/g or less, as measured in accordance with EDANA standard WSP 241.3.

Further, the above-described superabsorbent polymer may have a maximum strain of 0.30% to 1.50% and a recovery rate of 70% to 100% according to a creep test. A detailed method of the creep test will be described in Examples below. Preferably, the above-described superabsorbent polymer may have a maximum strain of 0.31% or more, 0.32% or more, 0.33% or more, 0.34% or more, 0.35% or more, 0.36% or more, 0.37 or more, 0.38% or more, 0.39% or more, 0.40% or more, 0.41% or more, 0.42% or more, or 0.43% or more, and 1.45% or less, 1.40% or less, 1.35% or less, 1.30% or less, 1.25% or less, or 1.23% or less according to the creep test. Preferably, the above-described superabsorbent polymer may have a recovery rate of 71% or more, 72% or more, 73% or more, 74% or more, 75% or more, 76% or more, 77% or more, 78% or more, 79% or more, or 80% or more according to the creep test.

Further, the above-described superabsorbent polymer may have a gel strength of 1500 Pa to 5000 Pa. A detailed method of measuring the gel strength will be described in Examples below. Preferably, the above-described superabsorbent polymer may have a gel strength of 1600 Pa or more, 1617 Pa or more, 1700 Pa or more, 1800 Pa or more, 1900 Pa or more, or 2000 Pa or more, and 4900 Pa or less, 4800 Pa or less, 4700 Pa or less, 4600 Pa or less, 4545 Pa or less, 4500 Pa or less, 4400 Pa or less, 4399 Pa or less, 4300 Pa or less, 4200 Pa or less, 4100 Pa or less, 4000 Pa or less, 3900 Pa or less, 3899 Pa or less, or 3800 Pa or less.

Further, the above-described superabsorbent polymer may have a permeability of 70 seconds to 150 seconds. A detailed method of measuring the permeability will be described in Examples below. Preferably, the above-described superabsorbent polymer may have a permeability of 71 seconds or more, 72 seconds or more, 73 seconds or more, 74 seconds or more, 75 seconds or more, 76 seconds or more, 77 seconds or more, 78 seconds or more, 79 seconds or more, 80 seconds or more, 81 seconds or more, 82 seconds or more, 83 seconds or more, or 84 seconds or more, and 145 seconds or less, 140 seconds or less, 135 seconds or less, 130 seconds or less, 125 seconds or less, 120 seconds or less, 115 seconds or less, 110 seconds or less, 105 seconds or less, or 100 seconds or less.

Further, the superabsorbent polymer may exhibit antimicrobial properties against all of the Gram-negative

bacteria and the Gram-positive bacteria. In this regard, the Gram-negative bacteria and the Gram-positive bacteria, on which the superabsorbent polymer exhibits antimicrobial properties, may be *Proteus Mirabilis* or *Escherichia coli,* and *Enterococcus faecalis,* respectively, but are not limited thereto.

**Method of preparing superabsorbent polymer**

Meanwhile, the superabsorbent polymer may be prepared by the following preparation method including the steps of:

forming a water-containing gel polymer by performing a crosslinking polymerization of an acrylic acid-based monomer including acidic groups, of which at least a part is neutralized, and a polymeric antimicrobial monomer represented by Chemical Formula 1 in the presence of an internal crosslinking agent and a polymerization initiator; and
forming a superabsorbent polymer including a crosslinked polymer by drying, pulverizing, and size-sorting the water-containing gel polymer.

The superabsorbent polymer prepared by the method may exhibit a 30 min-centrifuge retention capacity (CRC) of 29 g/g to 50 g/g for physiological saline (0.9 wt% aqueous sodium chloride solution), as measured in accordance with EDANA standard WSP 241.3 while exhibiting an antimicrobial property against at least one of Gram-positive bacteria and Gram-positive bacteria, as described above.

First, the step 1 is a step of forming a water-containing gel polymer by performing a crosslinking polymerization of an acrylic acid-based monomer including acidic groups, of which at least a part is neutralized, and a polymeric antimicrobial monomer in the presence of an internal crosslinking agent and a polymerization initiator.

The step may consist of a step of preparing a monomer composition by mixing the acrylic acid-based monomer, the internal crosslinking agent, and the polymerization initiator, and a step of forming the water-containing gel polymer by performing thermal polymerization or photopolymerization of the monomer composition. In this regard, for descriptions of the acrylic acid-based monomer and the internal crosslinking agent, reference may be made to those described above.

In the monomer composition, the internal crosslinking agent is included in an amount of 0.01 part by weight to 1 part by weight with respect to 100 parts by weight of the acrylic acid-based monomer, thereby crosslinking the polymerized polymer. When the amount of the internal crosslinking agent is less than 0.01 part by weight, the improvement effect due to crosslinking is insignificant. When the amount of the internal crosslinking agent is more than 1 part by weight, absorbency of the superabsorbent polymer may be reduced. More specifically, the internal crosslinking agent may be included in an amount of 0.05 parts by weight or more, or 0.1 part by weight or more, and 0.5 parts by weight or less, or 0.3 parts by weight or less with respect to 100 parts by weight of the acrylic acid-based monomer.

Further, the polymerization initiator may be appropriately selected depending on a polymerization method. When a thermal polymerization method is used, a thermal polymerization initiator is used. When a photopolymerization method is used, a photopolymerization initiator is used. When a hybrid polymerization method (a method of using both heat and light) is used, both the thermal polymerization initiator and the photopolymerization initiator are used. However, even though the photopolymerization method is used, a certain amount of heat is generated by light irradiation such as UV irradiation, etc., and is also generated with exothermic polymerization reaction. Therefore, the thermal polymerization initiator may be further used.

As the photopolymerization initiator, any compound capable of forming radicals by a light such as UV may be used without limitations in the constitution.

For example, one or more selected from the group consisting of benzoin ether, dialkyl acetophenone, hydroxyl alkylketone, phenyl glyoxylate, benzyl dimethyl ketal, acyl phosphine, and $\alpha$-aminoketone may be used as the photopolymerization initiator. Meanwhile, specific examples of acyl phosphine may include diphenyl(2,4,6-trimethylbenzoyl)phosphine oxide, phenylbis(2,4,6-trimethylbenzoyl)phosphine oxide, ethyl (2,4,6-trimethylbenzoyl) phenylphosphinate, etc. More various photopolymerization initiators are well disclosed in "UV Coatings: Basics, Recent Developments and New Application (Elsevier, 2007)" written by Reinhold Schwalm, p115, however, they are not limited to the above described examples.

The photopolymerization initiator may be included in an amount of 0.001 part by weight to 1 part by weight with respect to 100 parts by weight of the acrylic acid-based monomer. When the amount of the photopolymerization initiator is less than 0.001 part by weight, the polymerization rate may become slow, and when the amount of the photopolymerization initiator is more than 1 part by weight, a molecular weight of the superabsorbent polymer becomes small and its physical properties may become uneven. More specifically, the photopolymerization initiator may be included in an amount of 0.005 parts by weight or more, or 0.01 part by weight or more, or 0.1 part by weight or more, and 0.5 parts by weight or less, or 0.3 parts by weight or less with respect to 100 parts by weight of the acrylic acid-based monomer.

Further, when the thermal polymerization initiator is further included as the polymerization initiator, one or more selected from the group consisting of a persulfate-based initiator, an azo-based initiator, hydrogen peroxide, and ascorbic

acid may be used as the thermal polymerization initiator. Specific examples of the persulfate-based initiator may include sodium persulfate ($Na_2S_2O_8$), potassium persulfate ($K_2S_2O_8$), ammonium persulfate ($(NH_4)_2S_2O_8$), etc., and examples of the azo-based initiator may include 2,2-azobis-(2-amidinopropane)dihydrochloride, 2,2-azobis-(N,N-dimethylene)isobutyramidine dihydrochloride, 2-(carbamoylazo)isobutyronitrile, 2,2-azobis[2-(2-imidazolin-2-yl)propane]dihydrochloride, 4,4-azobis-(4-cyanovaleric acid), etc. More various thermal polymerization initiators are well disclosed in 'Principle of Polymerization(Wiley, 1981)', written by Odian, p 203, however, the thermal polymerization initiator is not limited to the above-described examples.

The thermal polymerization initiator may be included in an amount of 0.001 part by weight to 1 part by weight with respect to 100 parts by weight of the acrylic acid-based monomer. When the amount of the thermal polymerization initiator is less than 0.001 part by weight, additional thermal polymerization hardly occurs, and thus the addition effect of the thermal polymerization initiator may be insignificant. When the amount of the thermal polymerization initiator is more than 1 part by weight, the molecular weight of the superabsorbent polymer may become low and its physical properties may become uneven. More specifically, the thermal polymerization initiator may be included in an amount of 0.005 parts by weight or more, or 0.01 part by weight or more, or 0.1 part by weight or more, and 0.5 parts by weight or less, or 0.3 parts by weight or less with respect to 100 parts by weight of the acrylic acid-based monomer.

In addition to the polymerization initiators, the monomer composition may further include one or more kinds of additives such as a surfactant, a thickener, a plasticizer, a preservation stabilizer, an antioxidant, etc., if necessary, during crosslinking polymerization.

The above-described monomer composition including the acrylic acid-based monomer, the polymeric antimicrobial monomer, and the internal crosslinking agent, and optionally, the photopolymerization initiator, and the additives may be in the form of being dissolved in a solvent.

As the solvent to be applicable, any solvent may be used without limitations in view of constitution as long as it is able to dissolve the above components, and for example, one or more selected from water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethylether, toluene, xylene, butyrolactone, carbitol, methyl cellosolve acetate, and N,N-dimethylacetamide may be used in combination. The solvent may be included in the remaining amount excluding the above-described components, with respect to the total amount of the monomer composition.

Further, when a water-soluble solvent such as water is used as the solvent and a terpene-based compound having no solubility for water is used as the polymeric antimicrobial monomer, a surfactant may be additionally added in the amount of 10 parts by weight or less with respect to 100 parts by weight of the polymeric antimicrobial monomer in order to increase the solubility.

Meanwhile, the method of forming the water-containing gel polymer by photopolymerization of the monomer composition is also not particularly limited in view of constitution, as long as it is a polymerization method commonly used.

Specifically, the photopolymerization may be performed by irradiating UV with an intensity of 3 mW to 30 mW, or 10 mW to 20 mW at a temperature of 60°C to 90°C, or 70°C to 80°C. When the photopolymerization may be performed under these conditions, it is possible to form the crosslinked polymer with higher polymerization efficiency.

Further, when the photopolymerization is performed, it may be performed in a reactor equipped with a movable conveyor belt, but the above-described polymerization method is an example only, and the present disclosure is not limited to the above-described polymerization methods.

Further, as described above, when the photopolymerization is performed in a reactor equipped with a movable conveyor belt, the obtained water-containing gel polymer may be usually a sheet-like water-containing gel polymer having a width of the belt. In this case, a thickness of the polymer sheet may vary depending on the concentration of the monomer composition fed thereto and the feeding speed, and usually, it is preferable to supply the monomer composition such that a sheet-like polymer having a thickness of about 0.5 cm to about 5 cm may be obtained. When the monomer composition is supplied to such an extent that the thickness of the sheet-like polymer becomes too thin, it is undesirable because the production efficiency is low, and when the thickness of the sheet-like polymer is more than 5 cm, the polymerization reaction may not evenly occur over the entire thickness because of the excessive thickness.

Further, a water content of the water-containing gel polymer obtained by the above-mentioned method may be about 40% by weight to about 80% by weight with respect to the total weight of the water-containing gel polymer. Meanwhile, the "water content" as used herein means a weight occupied by water with respect to the total weight of the water-containing gel polymer, which may be a value obtained by subtracting the weight of the dried polymer from the weight of the water-containing gel polymer. Specifically, the water content may be defined as a value calculated by measuring the weight loss due to evaporation of moisture in the polymer during a process of drying by raising the temperature of the polymer through infrared heating. At this time, the water content is measured under the following drying conditions: the temperature is increased from room temperature to about 180°C and then the temperature is maintained at 180°C, and the total drying time is set to 20 minutes, including 5 minutes for the temperature rising step.

Meanwhile, after the preparation of the water-containing gel polymer, a process of coarsely pulverizing the prepared water-containing gel polymer may be optionally performed, before subsequent drying and pulverizing processes.

The coarse pulverization process is a process for increasing the drying efficiency in the subsequent drying process and controlling the particle size of the produced superabsorbent polymer powder. In this regard, a pulverizer used here is not limited by its configuration, and specifically, it may include any one selected from the group consisting of a vertical pulverizer, a turbo cutter, a turbo grinder, a rotary cutter mill, a cutter mill, a disc mill, a shred crusher, a crusher, a meat chopper, and a disc cutter, but is not limited to the above-described examples.

The coarse pulverization process may be performed, for example, such that the water-containing gel polymer may have a particle size of about 2 mm to about 10 mm. Pulverization of the water-containing gel polymer to a particle size of smaller than 2 mm is not technically easy due to the high water content of the water-containing gel polymer, and an agglomeration phenomenon between the pulverized particles may occur. Meanwhile, when the water-containing gel polymer is pulverized to a particle size of larger than 10 mm, the effect of increasing the efficiency in the subsequent drying step may be insignificant.

Meanwhile, after the preparation of the water-containing gel polymer, a process of coarsely pulverizing the prepared water-containing gel polymer may be optionally performed, before subsequent drying and pulverizing processes.

The coarse pulverization process is a process for increasing the drying efficiency in the subsequent drying process and controlling the particle size of the final produced superabsorbent polymer powder. In this regard, a pulverizer used here is not limited by its configuration, and specifically, it may include any one selected from the group consisting of a vertical pulverizer, a turbo cutter, a turbo grinder, a rotary cutter mill, a cutter mill, a disc mill, a shred crusher, a crusher, a meat chopper, and a disc cutter, but is not limited to the above-described examples.

The coarse pulverization process may be performed, for example, such that the water-containing gel polymer may have a particle size of about 2 mm to about 10 mm. Pulverization of the water-containing gel polymer to a particle size of smaller than 2 mm is not technically easy due to the high water content of the water-containing gel polymer, and an agglomeration phenomenon between the pulverized particles may occur. Meanwhile, when the water-containing gel polymer is pulverized to a particle size of larger than 10 mm, the effect of increasing the efficiency in the subsequent drying step may be insignificant.

Next, the step 2 is a step of forming a superabsorbent polymer including a crosslinked polymer by drying, pulverizing, and size-sorting the water-containing gel polymer prepared in the step 1.

The drying method may be selected and used without limitation in view of constitution, as long as it is commonly used in the process of drying the water-containing gel polymer. Specifically, the drying step may be performed by a method such as hot air supply, infrared irradiation, microwave irradiation, ultraviolet irradiation, etc.

Specifically, the drying may be performed at a temperature of about 150°C to about 250°C. When the drying temperature is lower than 150°C, the drying time becomes too long and the physical properties of the superabsorbent polymer finally formed may be deteriorated. When the drying temperature is higher than 250°C, only the polymer surface is excessively dried, and thus fine particles may be generated during the subsequent pulverization process and the physical properties of the superabsorbent polymer finally formed may be deteriorated. Therefore, the drying may be preferably performed at a temperature of 150°C or higher, or 160°C higher, and 200°C or lower, or 180°C or lower.

Meanwhile, the drying time may be about 20 min to about 90 min, in consideration of the process efficiency, but is not limited thereto.

The polymer after such a drying step may have a water content of about 5% by weight to about 10% by weight.

After the drying process, a pulverization process is performed.

The pulverization process may be performed such that the polymer powder, i.e., the superabsorbent polymer has a particle size of about 150 μm to about 850 μm. A pulverizer which is used for pulverization to such a particle size may include specifically a pin mill, a hammer mill, a screw mill, a roll mill, a disc mill, a jog mill, etc., but the present invention is not limited to the above-descried examples.

After the above pulverization step, to manage the physical properties of the superabsorbent polymer powder to be finally manufactured, the pulverized polymer powder may be further subjected to a size-sorting process according to the particle size.

Further, polymers having a particle size of about 150 μm to about 850 μm are sorted. Only a polymer having this particle size may be applied as a base polymer to a surface crosslinking reaction step, and finally commercialized.

The superabsorbent polymer resulting from the above process may have a fine powder form including the crosslinked polymer obtained by crosslinking polymerizing the acrylic acid-based monomer and the polymeric antimicrobial monomer via the internal crosslinking agent. Specifically, the superabsorbent polymer may have a fine powder form having a particle size of 150 μm to 850 μm.

Next, the step of performing surface crosslinking of the superabsorbent polymer prepared in the step 2 by heat treatment in the presence of a surface crosslinking agent may be further included.

The surface crosslinking is a step of increasing the crosslinking density in the vicinity of the surface of the superabsorbent polymer in connection with the internal crosslinking density of particles. In general, the surface crosslinking agent is applied to the surface of the polymer. Therefore, this reaction occurs on the surface of the polymer particle, which improves the crosslinking property on the surface of the particle without substantially affecting the interior of the particle. Thus, the surface-crosslinked superabsorbent polymer has a higher level of crosslinking in the vicinity of the surface than in the interior.

Such a surface crosslinking agent may be used in an amount of about 0.001 part by weight to about 5 parts by weight with respect to 100 parts by weight of the superabsorbent polymer. For example, the surface crosslinking agent may be used in an amount of about 0.005 parts by weight or more, 0.01 part by weight or more, or 0.05 parts by weight or more, and 5 parts by weight or less, 4 parts by weight or less, or 3 parts by weight or less with respect to 100 parts by weight of the superabsorbent polymer. It is possible to prepare a superabsorbent polymer exhibiting excellent general absorption properties by controlling the amount of the surface crosslinking agent in the above-described range.

Further, a method of mixing the surface crosslinking agent with the superabsorbent polymer is not limited in view of its construction. For example, a method of feeding the surface crosslinking agent and the superabsorbent polymer to a reactor and mixing them with each other, a method of spraying the surface crosslinking agent onto the superabsorbent polymer, or a method of mixing the superabsorbent polymer and the surface crosslinking agent while continuously feeding them to a mixer which is continuously operated may be used.

In addition to the surface crosslinking agent, water and alcohol are additionally mixed together to be added in the form of a surface crosslinking solution. When water and alcohol are added, there is an advantage in that the surface crosslinking agent may be uniformly dispersed in the superabsorbent polymer powder. Here, water and alcohol may be added in an amount of about 5 parts by weight to about 12 parts by weight with respect to 100 parts by weight of the polymer for the purpose of inducing uniform dispersion of the surface crosslinking agent, preventing agglomeration of the superabsorbent polymer powder, and optimizing the surface penetration depth of the crosslinking agent at the same time.

The surface crosslinking reaction may be carried out by heating the superabsorbent polymer powder, to which the surface crosslinking agent has been added, at a temperature of about 80°C to about 220°C for about 15 min to about 100 min. When the crosslinking reaction temperature is lower than 80°C, a sufficient surface crosslinking reaction may not occur, and when the crosslinking reaction temperature is higher than 220°C, an excessive surface crosslinking reaction may occur. Further, when the crosslinking reaction time is as short as less than 15 minutes, a sufficient crosslinking reaction may not occur, and when the crosslinking reaction time exceeds 100 minutes, the crosslinking density of the particle surface is excessively increased due to excessive surface crosslinking reaction, leading to deterioration in the physical properties. More specifically, the surface crosslinking reaction may be carried out by heating at a temperature of 120°C or higher, or 140°C or higher, and 200°C or lower, or 180°C or lower for 20 min or more, or 40 min or more, and 70 min or less, or 60 min or less.

A means for raising the temperature for the additional crosslinking reaction is not particularly limited. Heating may be performed by providing a heating medium or by directly providing a heat source. In this regard, the type of the heating medium applicable may be a hot fluid such as steam, hot air, hot oil, or the like. However, the present invention is not limited thereto. The temperature of the heating medium provided may be properly controlled, considering the means of the heating medium, the heating rate, and the target temperature. Meanwhile, as the heat source provided directly, an electric heater or a gas heater may be used, but the present invention is not limited to these examples.

Meanwhile, provided is a composition including the above-described superabsorbent polymer.

Furthermore, provided is an article including the above-described superabsorbent polymer.

The article may be one or more selected from absorbent articles, hygiene products, water retaining soil products, water stop materials for the civil engineering and construction, sheets for raising seedling, fresh-keeping agents, materials for poultice, electrical insulators, and articles for oral cavity, teeth, cosmetics, or skin.

In this regard, the hygiene products including the superabsorbent polymer may include, for example, hygiene products such as paper diapers for children, diapers for adults, sanitary pads, etc. In particular, the superabsorbent polymer may be preferably applied to diapers for adults, in which secondary odors due to bacterial growth are particularly problematic. Such hygiene products may have the configuration of common hygiene products, except that the above-described superabsorbent polymer of one embodiment is included in an absorber.

Hereinafter, preferred embodiments are provided for better understanding of the present invention. However, the following embodiments are only for illustrating the present invention, and the present invention is not limited thereto.

**[Preparation Example]**

Preparation Example A: Preparation of polymeric antimicrobial monomer 1-1

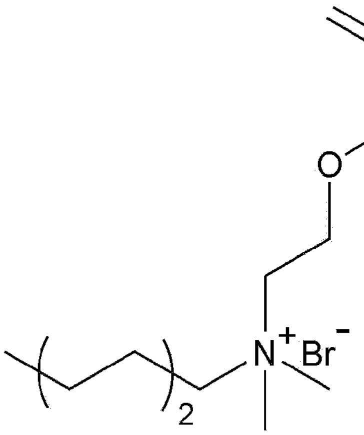

Acetonitrile (30 ml), 2-(dimethylamino)ethyl methacrylate (0.05 mol), bromohexane (0.05 mol), and p-methoxyphenol (4 mg) were put in a 250 ml flask. Thereafter, a reaction for preparing a quaternary ammonium salt by substituting an alkyl group in the amino group was carried out under stirring using a magnetic bar at 45°C for 24 hours. After 24 hours, the solution in which the reaction was completed was added to a diethyl ether solution (200 ml) to perform extraction. Then, the reaction product was filtered using a vacuum filter, and the remaining diethyl ether was completely removed to prepare a polymeric antimicrobial monomer 1-1 (15 g, yield: 75% or more).

MS$[M+H]^+$ = 322
$^1$H NMR (500MHz, DMSO-$d_6$,$\delta$[ppm]): 6.07, 5.76($R_2$,$R_3$), 1.90($R_1$), 4.51, 3.70, 3.69(L), 3.09($R_4$,$R_6$), 1.26, 0.87($R_5$)

Preparation Example B: Preparation of polymeric antimicrobial monomer 1-2

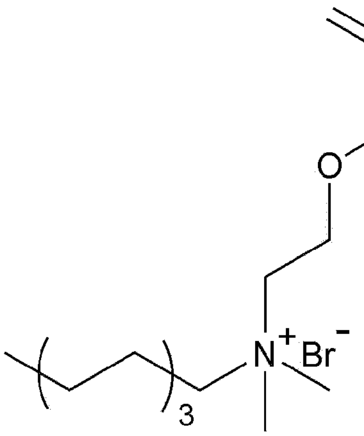

A polymeric antimicrobial monomer 1-2 (15 g, yield: 75% or more) was prepared in the same manner as in Preparation Example A, except that bromooctane was used instead of bromohexane in Preparation Example A.

MS$[M+H]^+$ = 350
1H NMR (500MHz, DMSO-$d_6$,$\delta$[ppm]): 6.07, 5.76($R_2$,$R_3$), 1.90($R_1$), 4.51, 3.70, 3.69(L), 3.09($R_4$,$R_6$), 1.26, 0.87($R_5$)]

Preparation Example C: Preparation of polymeric antimicrobial monomer 1-3

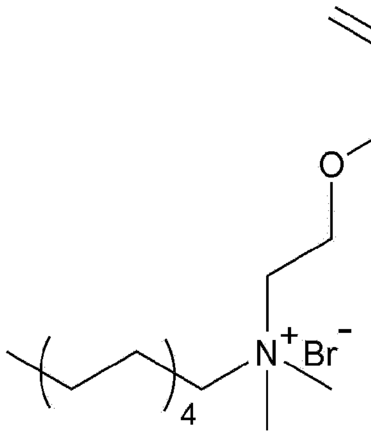

A polymeric antimicrobial monomer 1-3 (15 g, yield: 75% or more) was prepared in the same manner as in Preparation Example A, except that bromodecane was used instead of bromohexane in Preparation Example A.

MS$[M+H]^+$ = 378
1H NMR (500MHz, DMSO-$d_6$,$\delta$[ppm]): 6.07, 5.76($R_2$,$R_3$), 1.90($R_1$), 4.51, 3.70, 3.69(L), 3.09($R_4$,$R_6$), 1.26, 0.87($R_5$)

Preparation Example D: Preparation of polymeric antimicrobial monomer 1-4

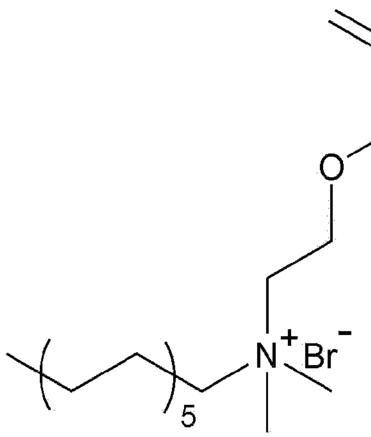

A polymeric antimicrobial monomer 1-4 (15 g, yield: 75% or more) was prepared in the same manner as in Preparation Example A, except that bromododecane was used instead of bromohexane in Preparation Example A.

MS$[M+H]^+$ = 406
1H NMR (500MHz, DMSO-$d_6$,$\delta$[ppm]): 6.07, 5.76($R_2$,$R_3$), 1.90($R_1$), 4.51, 3.70, 3.69(L), 3.09($R_4$,$R_6$), 1.26, 0.87($R_5$)

**[Examples and Comparative Examples]**

**Example 1**

Acrylic acid (100 g), polyethylene glycol diacrylate (Mn=575, 0.23 g) as an internal crosslinking agent, bis(2,4,6-trimethylbenzoyl)-phenylphosphine oxide (0.008 g) as a photoinitiator, sodium persulfate (SPS, 0.12 g) as a thermal initiator, a 98% sodium hydroxide solution (39.7 g), and the polymeric antimicrobial monomer 1-1 (1 g) prepared in Preparation Example A were put in a 3 L glass container equipped with a stirrer, a nitrogen injector, and a thermometer, and a water-soluble unsaturated monomer aqueous solution was prepared while continuously adding nitrogen thereto.

The aqueous solution of the water-soluble unsaturated monomer was added to a stainless steel container with a width of 250 mm, a length of 250 mm, and a height of 30 mm, and irradiated with ultraviolet rays (irradiation dose: 10 $mV/cm^2$) in a UV chamber at 80°C for 60 seconds and aged for 2 minutes to obtain a water-containing gel polymer.

The obtained water-containing gel polymer was pulverized into a size of 3 mm * 3 mm, and then the obtained gel-type resin was spread to a thickness of about 30 mm on a stainless wire gauze having a hole size of 600 $\mu$m, and

dried in a hot air oven at 120°C for 10 hours. The dried polymer thus obtained was pulverized using a pulverizer, and sieved using an ASTM standard sieve to obtain a base polymer having a particle size of 300 μm to 600 μm, which was determined as a superabsorbent polymer.

**Example 2**

A superabsorbent polymer was prepared in the same manner as in Example 1, except that the polymeric antimicrobial monomer 1-2 prepared in Preparation Example B, instead of the polymeric antimicrobial monomer 1-1 prepared in Preparation Example A, was used in Example 1.

**Example 3-1**

A superabsorbent polymer was prepared in the same manner as in Example 1, except that the polymeric antimicrobial monomer 1-3 prepared in Preparation Example C, instead of the polymeric antimicrobial monomer 1-1 prepared in Preparation Example A, was used in Example 1.

**Example 3-2**

A superabsorbent polymer was prepared in the same manner as in Example 1, except that 0.1 g of the polymeric antimicrobial monomer 1-3 prepared in Preparation Example C was used in Example 3-1.

**Example 3-3**

A superabsorbent polymer was prepared in the same manner as in Example 1, except that 10 g of the polymeric antimicrobial monomer 1-3 prepared in Preparation Example C was used in Example 3-1.

**Example 3-4**

A superabsorbent polymer was prepared in the same manner as in Example 1, except that 20 g of the polymeric antimicrobial monomer 1-3 prepared in Preparation Example C was used in Example 3-1.

**Example 4-1**

A superabsorbent polymer was prepared in the same manner as in Example 1, except that the polymeric antimicrobial monomer 1-4 prepared in Preparation Example D, instead of the polymeric antimicrobial monomer 1-1 prepared in Preparation Example A, was used in Example 1.

**Example 4-2**

A superabsorbent polymer was prepared in the same manner as in Example 1, except that 0.5 g of the polymeric antimicrobial monomer 1-4 prepared in Preparation Example D was used in Example 4-1.

**Example 4-3**

A superabsorbent polymer was prepared in the same manner as in Example 1, except that 2 g of the polymeric antimicrobial monomer 1-4 prepared in Preparation Example D was used in Example 4-1.

**Example 4-4**

A superabsorbent polymer was prepared in the same manner as in Example 1, except that 10 g of the polymeric antimicrobial monomer 1-4 prepared in Preparation Example D was used in Example 4-1.

**Comparative Example 1**

A superabsorbent polymer was prepared in the same manner as in Example 1, except that no antimicrobial monomer was used in Example 1.

**Comparative Example 2**

A superabsorbent polymer was prepared in the same manner as in Example 1, except that 0.01 g of the polymeric antimicrobial monomer 1-3 prepared in Preparation Example C was used in Example 3-1.

**Comparative Example 3**

A superabsorbent polymer was prepared in the same manner as in Example 1, except that 25 g of the polymeric antimicrobial monomer 1-3 prepared in Preparation Example C was used in Example 3-1.

**Comparative Example 4**

9.9 g of the superabsorbent polymer prepared in Comparative Example 1 and 0.1 g of the antimicrobial monomer 1-4 prepared in Preparation Example D were simply mixed (mixed in the same proportion as the antimicrobial monomer used in the superabsorbent polymer prepared in Example 4-1).

**[Experimental Example 1]**

Physical properties of the superabsorbent polymers prepared in Examples and Comparative Examples were evaluated by the following methods, and the results are shown in Tables 1 and 2 below. Unless otherwise indicated, all the following physical properties were evaluated at a constant temperature and a constant humidity (23±1°C, relative humidity of 50±10%), and a saline solution or brine means an aqueous solution of 0.9 wt% sodium chloride (NaCl).

**(1) Centrifuge retention capacity (CRC)**

Centrifuge retention capacity by absorption rate under no load was measured for the superabsorbent polymers of Examples and Comparative Examples according to European Disposables and Nonwovens Association (EDANA) standard WSP 241.3, respectively.

In detail, Wr(g) (about 0.2 g) of the superabsorbent polymer was uniformly put in a bag made of non-woven fabric and sealed, and then, soaked in a saline solution (0.9 wt% aqueous sodium chloride solution) at room temperature. After 30 minutes, it was drained for 3 minutes under 250G using a centrifuge, and the weight $W_2$(g) of the bag was measured. Further, the same operation was conducted without using the polymer, and then the weight $W_1$(g) at that time was measured. Using each the obtained weight, CRC(g/g) was calculated according to the following Equation. The results are shown in Table 1 below.

[Mathematical Equation 1]

$$CRC\ (g/g) = \{[W_2(g) - W_1(g)]/W_0(g)\} - 1$$

in Mathematical Equation 1,

Wr(g) represents the initial weight (g) of the superabsorbent polymer,
$W_1$(g) represents the weight of the bag without the superabsorbent polymer, which was allowed to absorb the physiological saline by immersing therein for 30 minutes, and then dehydrated using a centrifuge at 250 G for 3 minutes, and
$W_2$(g) represents the weight of the bag with the superabsorbent polymer, which was allowed to absorb the physiological saline by immersing therein at room temperature for 30 minutes, and then dehydrated using a centrifuge at 250 G for 3 minutes

**(2) Evaluation of antimicrobial property against *Proteus* Mirabilis**

2 g of each superabsorbent polymer prepared in Examples and Comparative Examples was put in a 250 cell culture flask, and 50 ml of artificial urine, to which a test bacterium, *Proteus Mirabilis* (ATCC 7002) was inoculated at 3000±300 CFU/ml, was injected thereto. Thereafter, to allow the superabsorbent polymer to sufficiently absorb the artificial urine solution, they were mixed for about 1 minute. When the polymer sufficiently absorbed the solution, it exhibited a gel form, which was incubated in an incubator (JEIO TECH) at 35°C for 12 hours. To the sample, of which incubation was completed, 150 ml of 0.9 wt% NaCl solution was added, followed by shaking for about 1 minute. This

dilution was spread on an agar medium plate. Thereafter, serial dilution was performed for colony counting, and in this procedure, 0.9 wt% NaCl solution was used. Antibacterial performance was determined by calculating an antibacterial rate (%) of *Proteus Mirabilis* (ATCC 7002) according to the following Mathematical Equation 1 after calculating the initial concentration of the bacteria (Co, CFU/ml) based on the dilution concentrations. The results are shown in Table 1 below.

[Mathematical Equation 1]

$$\text{Antibacterial rate (\%)} = \left(1 - \frac{C_{sample}}{C_{Refernce}}\right) \times 100$$

wherein $C_{sample}$ represents CFU of bacteria after incubation in a superabsorbent polymer with an antimicrobial material, and $C_{Reference}$ represents CFU of bacteria after incubation in a superabsorbent polymer of Comparative Example 1 without the antimicrobial material.

**(3) Evaluation of leakage of antimicrobial monomer**

To examine leakage of the antimicrobial monomer from the superabsorbent polymers prepared in Examples, the residual amount of the antimicrobial monomer was measured. In detail, 0.1 g of the prepared superabsorbent polymer and 20 ml of 0.9wt% saline solution were shaken for 1 hour, and then a liquid extract was filtered, and the amount of the leaked antimicrobial monomer was examined with UPLC/QDa. The results are shown in Table 1 below.

[Table 1]

| | Type of antimicrobi al monomer | Amount of antimicrobi al monomer1) | Mean Log CFU/ml | Antibacteri al rate (%) | CRC (g/g) | Amount of residual antimicrobial monomer in superabsorbent polymer (wt%) |
|---|---|---|---|---|---|---|
| Ex. 1 | 1-1 | 1.0 | 9.09 | 52.1 | 43.3 | N.D |
| Ex. 2 | 1-2 | 1.0 | 9.03 | 58.3 | 42.1 | N.D |
| Ex. 3-1 | 1-3 | 1.0 | 6.40 | 99.9 | 41.0 | N.D |
| Ex. 3-2 | 1-3 | 0.1 | 6.41 | 99.9 | 44.2 | N.D |
| Ex. 3-3 | 1-3 | 10.0 | 6.37 | 99.9 | 34.0 | N.D |
| Ex. 3-4 | 1-3 | 20.0 | 6.33 | 99.9 | 31.1 | N.D |
| Ex. 4-1 | 1-4 | 1.0 | 6.39 | 99.9 | 40.2 | N.D |
| Ex. 4-2 | 1-4 | 0.5 | 6.41 | 99.9 | 42.9 | N.D |
| Ex. 4-3 | 1-4 | 2.0 | 6.40 | 99.9 | 38.5 | N.D |
| Ex. 4-4 | 1-4 | 10.0 | 6.31 | 99.9 | 33.2 | N.D |
| Compara five Ex. 1 | - | - | 9.41 | - | 45.0 | - |
| Compara five Ex. 2 | 1-3 | 0.01 | 9.40 | 2.28 | 44.4 | - |
| Comparative Ex. 3 | 1-3 | 25.0 | - | 99.9 | 28.9 | - |
| Compara tive Ex. 4 | 1-4(simple mixing) | 2.0 | - | | | 3.43 |
| 1) parts by weight with respect to 100 parts by weight of acrylic acid monomer | | | | | | |

Referring to Table 1, it was confirmed that the superabsorbent polymers of Examples exhibited excellent antimicrobial property against *Proteus Mirabilis* which is one of Gram-negative bacteria while satisfying a 30 min-centrifuge retention capacity (CRC) of 29 g/g to 50 g/g for physiological saline (0.9 wt% aqueous sodium chloride solution), as

measured in accordance with EDANA standard WSP 241.3, unlike the superabsorbent polymers of Comparative Examples.

Further, the superabsorbent polymers of Examples showed no detection of residual antimicrobial monomers, unlike the superabsorbent polymer of Comparative Example 4, in which the antimicrobial monomer and the superabsorbent polymer were simply mixed. Therefore, it was confirmed that the superabsorbent polymers of Examples may continuously exhibit excellent antimicrobial property without leakage of the antimicrobial agent even over time.

**(4) Evaluation of antimicrobial property against *Escherichia coli***

To examine antimicrobial properties of the superabsorbent polymers prepared in Examples and Comparative Examples against *Escherichia coli,* their antibacterial rate(%) of *Escherichia coli* (E.coli, ATCC 25922) was calculated in the same manner as in evaluating the antimicrobial property against *Proteus Mirabilis,* except that artificial urine, to which *Escherichia coli* (E.coli, ATCC 25922) was inoculated at $10^5 \pm 1000$ CFU/ml, was used instead of the artificial urine to which *Proteus Mirabilis* (ATCC 7002) was inoculated at $3000 \pm 300$ CFU/ml. The results are shown in Table 2 below.

**(5) Evaluation of antimicrobial property against *Enterococcus faecalis***

To examine antimicrobial properties of the superabsorbent polymers prepared in Examples and Comparative Examples against *Enterococcus faecalis,* their antibacterial rate(%) of *Enterococcus faecalis* (*E.faecalis,* ATCC 29212) was calculated in the same manner as in evaluating the antimicrobial property against *Proteus Mirabilis,* except that artificial urine, to which *Enterococcus faecalis* (*E.faecalis,* ATCC 29212) was inoculated at $3000 \pm 300$ CFU/ml, was used instead of the artificial urine to which *Proteus Mirabilis* (ATCC 7002) was inoculated at $3000 \pm 300$ CFU/ml. The results are shown in Table 2 below.

[Table 2]

| | Type of antimicrobial monomer | Amount of antimicrobial monomer 1) | Type of bacteria | Mean Log CFU/ml | Antibacterial rate (%) |
|---|---|---|---|---|---|
| Ex. 3-1 | 1-3 | 1.0 | *Escherichia coli* | 6.45 | 99.9 |
| Ex. 4-1 | 1-4 | 1.0 | *Escherichia coli* | 6.46 | 99.9 |
| Ex. 3-1 | 1-3 | 1.0 | *Enterococcus faecalis* | 6.36 | 99.9 |
| Ex. 4-1 | 1-4 | 1.0 | *Enterococcus faecalis* | 6.40 | 99.9 |
| 1) parts by weight with respect to 100 parts by weight of acrylic acid monomer | | | | | |

Referring to Table 2, it was confirmed that the superabsorbent polymers of Examples also exhibited excellent antimicrobial properties against a Gram-negative bacterium, *Escherichia coli* and a Gram-positive bacterium, *Enterococcus faecalis.*

Accordingly, it was confirmed that the superabsorbent polymer including the crosslinked polymer prepared by using the antimicrobial monomer having a specific structure of a quaternary ammonium salt moiety may exhibit the water retention capacity above a predetermined level while exhibiting the antimicrobial property against both Gram-positive bacteria and Gram-negative bacteria.

**[Experimental Example 2]**

Rheological properties of the superabsorbent polymers according to the present invention were analyzed by the following methods.

**1) Test samples**

6 types of samples were used as follows:

#1: The superabsorbent polymer prepared in Example 3-1 was used as sample #1.

#2: 100 g of the superabsorbent polymer prepared in Example 3-1 was mixed with a solution containing a mixture of 3 g of water, 3 g of methanol, and 0.2 g of ethylene glycol diglycidyl ether using a high-speed mixer, and allowed to react at 140°C for 40 min, and then cooled to room temperature to prepare a superabsorbent polymer, which was used as sample #2.

#3: 100 g of the superabsorbent polymer prepared in Example 3-1 was mixed with a solution containing a mixture of 3 g of water, 3 g of methanol, and 0.2 g of 1,3-propanediol using a high-speed mixer, and allowed to react at 140°C for 40 min, and then cooled to room temperature to prepare a superabsorbent polymer, which was used as sample #3.

#4: A superabsorbent polymer was prepared in the same manner as in Example 3-1, and then 100 g of the prepared superabsorbent polymer was mixed with a solution containing a mixture of 3 g of water, 3 g of methanol, and 0.2 g of ethylene glycol diglycidyl ether using a high-speed mixer, and allowed to react at 140°C for 40 min, and then cooled to room temperature to prepare a superabsorbent polymer, which was used as sample #4.

#5: 100 g of the superabsorbent polymer prepared in Comparative Example 1 was mixed with a solution containing a mixture of 3 g of water, 3 g of methanol, and 0.2 g of ethylene glycol diglycidyl ether using a high-speed mixer, and allowed to react at 140°C for 40 min, and then cooled to room temperature to prepare a superabsorbent polymer, which was used as sample #6.

#6: BASF's single odor control sap (OC6600)

### 2) Creep Test

A force of 10 Pa was applied to the sample for 1 min to measure a strain, and then the force was removed for 2 min to measure a recovery rate, and the results are shown in FIG. 1 and Table 3 below.

[Table 3]

| Sample | Maximum strain (%) | Recovery rate(%) |
|---|---|---|
| #1 | 1.23 | 72 |
| #2 | 0.40 | 78 |
| #3 | 0.32 | 100 |
| #4 | 0.43 | 84 |
| #5 | 0.45 | 67 |
| #6 | 0.52 | 67 |

As shown in FIG. 1 and Table 3, the maximum strain after surface crosslinking (#2, #3, #4) was reduced, as compared with the maximum strain before surface crosslinking (#1), indicating that the maximum strain was reduced, as the elastic force increased due to formation of a core-shell structure. In addition, the superabsorbent polymers (#1, #2, #3, #4) according to the present invention all had a recovery rate of 70% or more, which was higher than those of general superabsorbent polymers (#5, #6). It can be seen that the wearing comfort may be maintained for a long time and the destruction probability of the structure of the superabsorbent polymer is low.

### 3) Frequency Sweep Test and Amplitude Sweep Test

A frequency sweep test was performed for the samples, and specifically, gel strength was measured as follows.

1 g of the sample was immersed and swollen in 100 g of physiological saline for 1 hr. Thereafter, the unabsorbed solvent was removed for 4 minutes using an aspirator, and the solvent on the surface was wiped off once by evenly distributing on the filter paper.

2.5 g of the swollen superabsorbent polymer sample was placed between a rheometer and two plates (25 mm in diameter, with a wall of about 2 mm at the bottom to prevent the sample from escaping), and the gap between the two plates was adjusted to 1 mm (At this time, when it was difficult to adjust the gap to 1 mm because the sample was hard, the plates were pressurized with a force of about 3 N and the gap between the plates was controlled so that the swollen superabsorbent polymer sample fully came in contact with the plates). Subsequently, the superabsorbent polymer sample between the plates was stabilized for about 5 min. Then, while increasing strain at a

frequency of 10 rad/s using the rheometer, strain was confirmed in the region of the linear viscoelastic regime where a storage modulus (G') and a loss modulus (G") were constant. In general, in the swollen superabsorbent polymer sample, there was 0.1% strain in the linear viscoelastic regime region. At a constant frequency of 10 rad/s, viscoelasticity (G', G") of the superabsorbent polymer swollen for 60 seconds under strain of the linear regime region were measured. The average value of G' was determined as a gel strength.

The results are shown in FIGS. 2 and 3 and Table 4.

[Table 4]

| Sample | Storage Modulus (Pa) @10rad/s |
|---|---|
| #1 | 1617 |
| #2 | 4399 |
| #3 | 3899 |
| #4 | 4545 |
| #5 | 3745 |
| #6 | 4318 |

As shown in FIG. 2 and Table 4, the gel strength after surface crosslinking (#2, #3, #4) was increased, as compared with the gel strength before surface crosslinking (#1), indicating formation of a core-shell structure. In addition, the superabsorbent polymers (#1, #2, #3, #4) according to the present invention showed the gel strength at a higher or similar level, as compared with general superabsorbent polymers (#5, #6), indicating that mechanical properties were similarly maintained or improved.

As shown in FIG. 3, a strain-overshoot behavior was observed in the loss modulus after surface crosslinking (#2, #3, #4), unlike that before surface crosslinking (#1), indicating formation of a core-shell structure due to surface crosslinking.

**4) Permeability**

Permeability was measured using a 0.9% saline solution under a load of 0.3 psi, as described in the literature (Buchholz, F.L. and Graham, A.T., "Modern Superabsorbent Polymer Technology," John Wiley & Sons(1998), page 161).

In more detail, 0.2 g of particles having a particle size of 300 μm to 600 μm were taken from each sample, and added to a cylinder (Φ20 mm), wherein the cylinder has a stopcock on one end, an upper limit mark and a lower limit mark thereon. The upper limit mark on the cylinder is indicated at the position where 40 ml of (saline) solution is filled into the cylinder, and the lower limit mark on the cylinder is indicated at the position where 20 ml of (saline) solution is filled into the cylinder.

50 g of 0.9% saline (NaCl) solution was added to the cylinder with the stopcock in a closed position, and left for 30 minutes. Then, if necessary, additional saline solution was added to the cylinder to bring the level of saline solution to the upper limit mark on the cylinder. Then, a load of 0.3 psi was applied to the cylinder including the saline-absorbed superabsorbent polymers, and left for 1 min. Thereafter, the stopcock at the bottom of the cylinder was open to measure the time taken for the 0.9% saline solution to pass from the upper limit mark to the lower limit mark on the cylinder. All measurements were carried out at a temperature of 24±1°C and relative humidity of 50±10%.

The time taken to pass from the upper limit mark to the lower limit mark was measured for sample($T_s$) and also measured in the absence of the superabsorbent polymer, and permeability was calculated by the following Equation 1. The results are shown in Table 5.

[Equation 1]

$$\text{Permeability (sec)} = T_S - T_0$$

[Table 5]

| Sample | Permeability(s) |
|---|---|
| #2 | 83 |
| #3 | 81 |
| #4 | 84 |
| #5 | 78 |

As shown in Table 5, it was confirmed that the superabsorbent polymers according to the present invention had permeability which is generally required for superabsorbent polymers.

## Claims

1. A superabsorbent polymer comprising a crosslinked polymer of an acrylic acid-based monomer including acidic groups, of which at least a part is neutralized, a polymeric antimicrobial monomer represented by the following Chemical Formula 1, and an internal crosslinking agent:

[Chemical Formula 1]

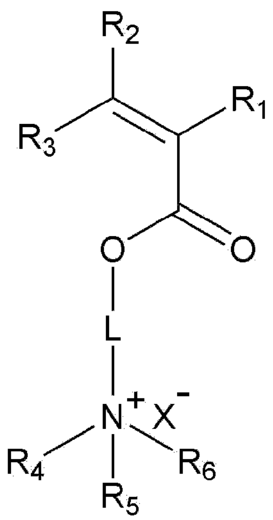

in Chemical Formula 1,

L is alkylene having 1 to 10 carbon atoms,
$R_1$ to $R_3$ are each independently hydrogen or methyl,
among $R_4$ to $R_6$, one is an alkyl having 6 to 20 carbon atoms, and the others are each independently an alkyl having 1 to 4 carbon atoms,
X is halogen.

2. The superabsorbent polymer of claim 1, wherein in Chemical Formula 1, L is methylene, ethylene, or propylene.

3. The superabsorbent polymer of claim 1, wherein in Chemical Formula 1, among $R_4$ to $R_6$, one is alkyl having 6 to 20 carbon atoms and the others are methyl.

4. The superabsorbent polymer of claim 1, wherein $R_1$ is hydrogen, or methyl, $R_2$ and $R_3$ are hydrogen, and among $R_4$ to $R_6$, one is alkyl having 10 to 20 carbon atoms and the others are methyl.

5. The superabsorbent polymer of claim 1, wherein the polymeric antimicrobial monomer is a compound represented by any one of the following Chemical Formulae 1-1 to 1-4:

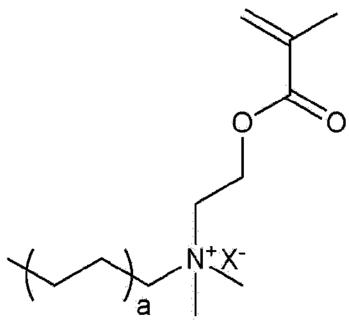

**1-1**

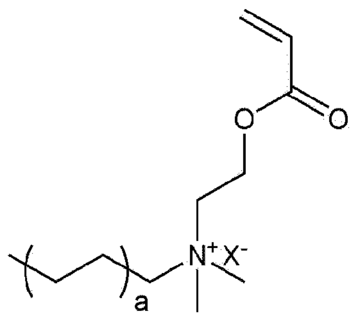

**1-2**

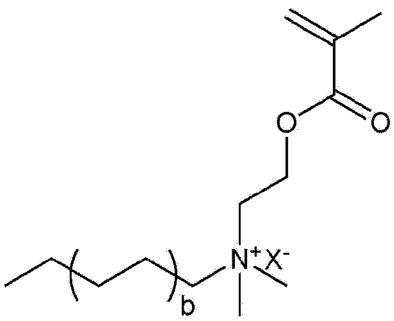

**1-3**

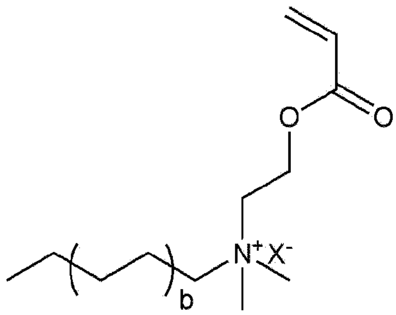

**1-4**

in Chemical Formulae 1-1 to 1-4,

a is an integer of 2 to 9,
b is an integer of 2 to 8, and
X is bromo or chloro.

6. The superabsorbent polymer of claim 1, wherein the polymeric antimicrobial monomer is included in the crosslinked polymer in an amount of 0.1 part by weight to 20 parts by weight with respect to 100 parts by weight of the acrylic acid-based monomer.

7. The superabsorbent polymer of claim 1, further comprising a surface-modified layer on the crosslinked polymer, wherein the surface-modified layer is prepared by additionally crosslinking the crosslinked polymer via a surface crosslinking agent.

8. The superabsorbent polymer of claim 1, wherein the superabsorbent polymer has a 30 min-centrifuge retention capacity (CRC) of 29 g/g to 50 g/g for physiological saline (0.9 wt% aqueous sodium chloride solution), as measured in accordance with EDANA standard WSP 241.3.

9. The superabsorbent polymer of claim 1, wherein the superabsorbent polymer has a maximum strain of 0.30% to 1.50% and a recovery rate of 70% to 100% according to a creep test.

10. The superabsorbent polymer of claim 1, wherein the superabsorbent polymer has a gel strength of 1500 Pa to 5000 Pa.

**11.** The superabsorbent polymer of claim 1, wherein
the superabsorbent polymer has a permeability of 70 seconds to 150 seconds.

**12.** The superabsorbent polymer of claim 1, wherein
the superabsorbent polymer has a core-shell structure.

**13.** The superabsorbent polymer of claim 1, wherein
the superabsorbent polymer exhibits an antimicrobial property against at least one of Gram-positive bacteria and Gram-negative bacteria.

**14.** The superabsorbent polymer of claim 13, wherein
the superabsorbent polymer exhibits an antimicrobial property against both Gram-positive bacteria and Gram-negative bacteria.

**15.** The superabsorbent polymer of claim 13, wherein
the Gram-negative bacteria is *Proteus Mirabilis* or *Escherichia coli,* and the Gram-positive bacteria is *Enterococcus faecalis.*

**16.** A method of preparing a superabsorbent polymer, the method comprising the steps of:

forming a water-containing gel polymer by performing a crosslinking polymerization of an acrylic acid-based monomer including acidic groups, of which at least a part is neutralized, and a polymeric antimicrobial monomer represented by Chemical Formula 1 in the presence of an internal crosslinking agent and a polymerization initiator; and
forming a superabsorbent polymer including a crosslinked polymer by drying, pulverizing, and size-sorting the water-containing gel polymer:

[Chemical Formula 1]

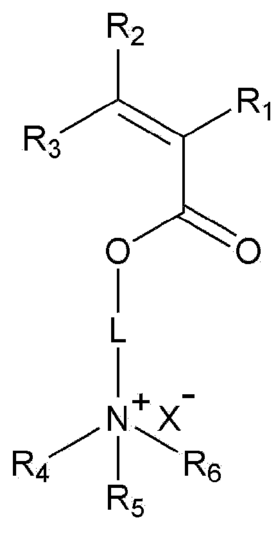

in Chemical Formula 1,

L is alkylene having 1 to 10 carbon atoms,
$R_1$ to $R_3$ are each independently hydrogen or methyl,
among $R_4$ to $R_6$, one is alkyl having 6 to 20 carbon atoms, and the others are each independently alkyl having 1 to 4 carbon atoms, and
X is halogen.

**17.** The method of claim 16, wherein
the polymeric antimicrobial monomer is used in an amount of 0.1 part by weight to 20 parts by weight with respect

to 100 parts by weight of the acrylic acid-based monomer.

**18.** The method of claim 16, further comprising the step of surface-crosslinking the superabsorbent polymer by heat treatment in the presence of a surface crosslinking agent.

**19.** The method of claim 16, wherein
the prepared superabsorbent polymer exhibits an antimicrobial property against at least one of Gram-positive bacteria and Gram-negative bacteria.

**20.** A composition comprising the superabsorbent polymer of any one of claims 1 to 15.

**21.** An article comprising the superabsorbent polymer of any one of claims 1 to 15.

[FIG. 1]

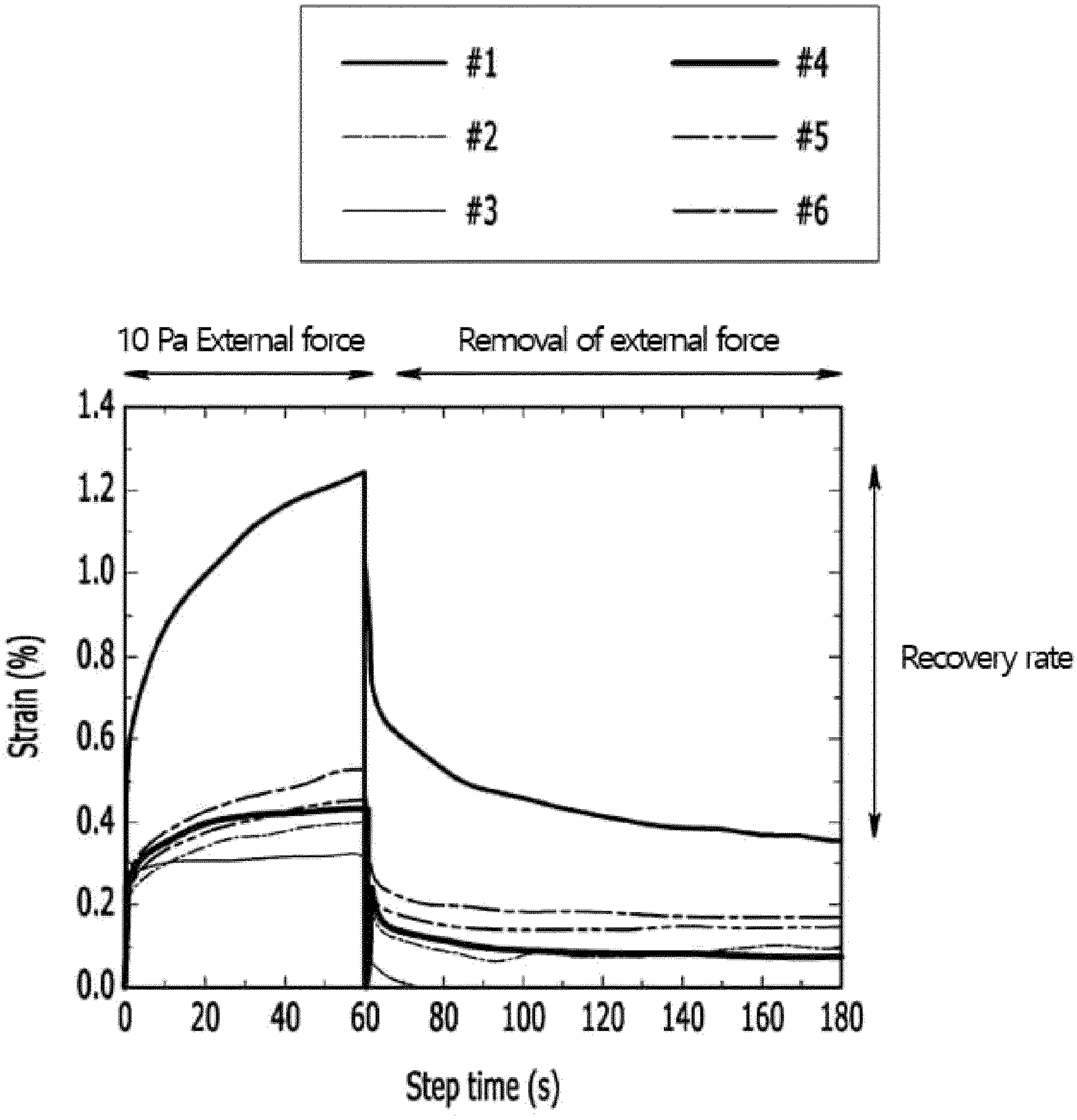

#1
#4
#2
#5
#3
#6
10 Pa External force
Removal of external force
1.4
1.2
1.0
0.8
0.6
0.4
0.2
0.0
Strain (%)
0
20
40
60
80
100
120
140
160
180
Step time (s)
Recovery rate

[FIG. 2]

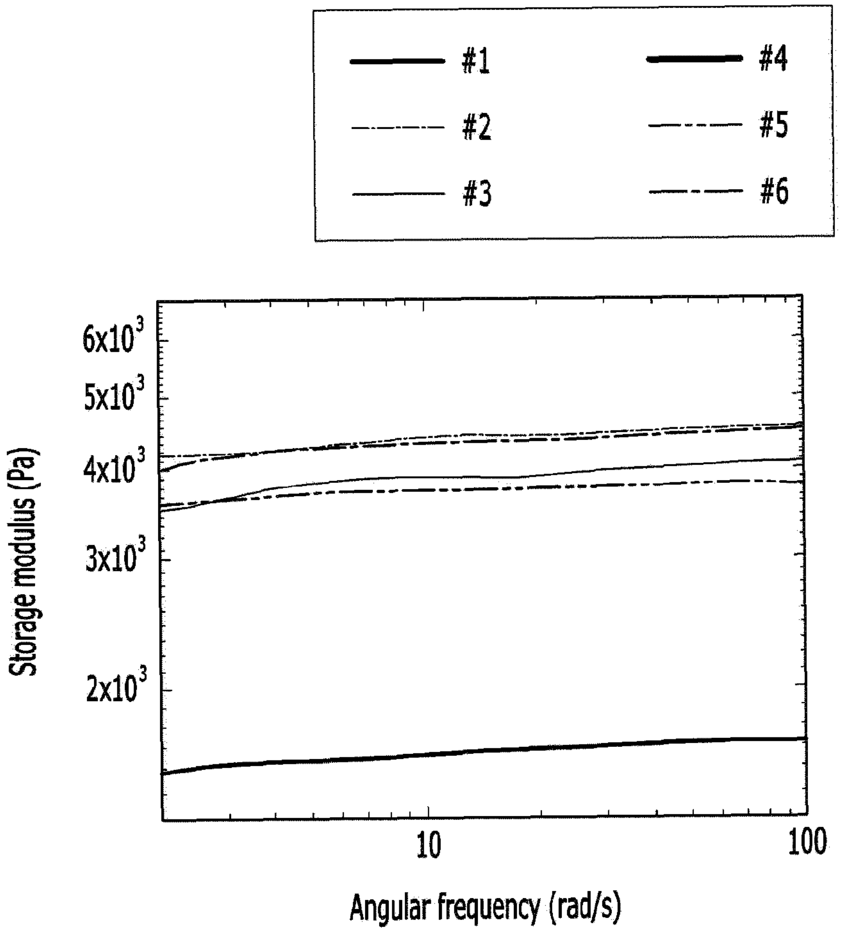
#1
#2
#3
#4
#5
#6
Storage modulus (Pa)
6x10^3
5x10^3
4x10^3
3x10^3
2x10^3
10
100
Angular frequency (rad/s)

[FIG. 3]

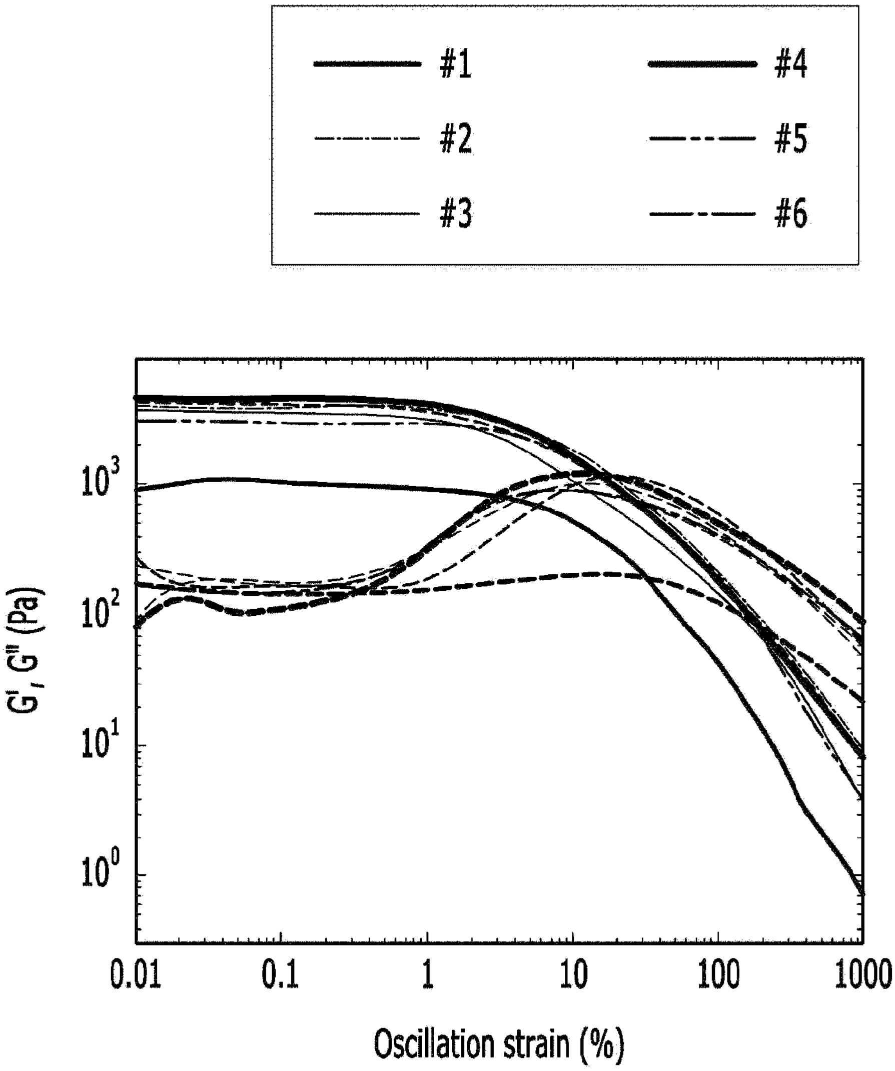
#1
#4
#2
#5
#3
#6
G', G" (Pa)
10^3
10^2
10^1
10^0
0.01
0.1
1
10
100
1000
Oscillation strain (%)

## INTERNATIONAL SEARCH REPORT

International application No.
**PCT/KR2021/013807**

### A. CLASSIFICATION OF SUBJECT MATTER

**C08F 220/34**(2006.01)i; **C08F 220/04**(2006.01)i; **C08J 3/24**(2006.01)i; **A61L 15/60**(2006.01)i

According to International Patent Classification (IPC) or to both national classification and IPC

### B. FIELDS SEARCHED

Minimum documentation searched (classification system followed by classification symbols)

C08F 220/34(2006.01); C08F 216/14(2006.01); C08F 220/06(2006.01); D01F 1/10(2006.01); D01F 6/92(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 고흡수(super-absorbent, SAP), 아크릴레이트(acrylate), 항균성(anti-microbial, anti-bacterial, anti-viral), 가교(crosslink), 암모늄염(ammonium salt)

### C. DOCUMENTS CONSIDERED TO BE RELEVANT

| Category* | Citation of document, with indication, where appropriate, of the relevant passages | Relevant to claim No. |
|---|---|---|
| Y | CN 108239215 A (WANHUA CHEMICAL GROUP CO., LTD.) 03 July 2018 (2018-07-03)<br>See claims 1-15; and paragraphs [0029] and [0045]-[0048]. | 1-21 |
| Y | XUE, Y. et al. Antimicrobial polymeric materials with quaternary ammonium and phosphonium salts. International journal of molecular sciences. 2015, vol. 16, no. 2, pp. 3626-3655.<br>See abstract; page 3636; and figure 5. | 1-21 |
| Y | LIU, X, et al. Modified acrylic-based superabsorbents with hydrophobic monomers: synthesis, characterization and swelling behaviors. Journal of Polymer Research. 2011, vol. 18, no. 5, pp. 897-905.<br>See abstract; and page 898. | 1-21 |
| Y | CN 108774759 A (HUA SICHENG NON-WOVEN FABRICS CO., LTD. OF JIANGYIN CITY) 09 November 2018 (2018-11-09)<br>See paragraph [0007]. | 1-21 |
| Y | LI, X. et al. Synthesis and water absorbency of polyampholytic hydrogels with antibacterial activity. Journal of applied polymer science. 2009, vol. 112, no. 1, pp. 439-446.<br>See abstract. | 1-21 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 January 2022** | **25 January 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**
**Information on patent family members**

International application No.
**PCT/KR2021/013807**

| Patent document cited in search report | Publication date (day/month/year) | Patent family member(s) | Publication date (day/month/year) |
|---|---|---|---|
| CN 108239215 A | 03 July 2018 | WO 2018-120056 A1 | 05 July 2018 |
| CN 108774759 A | 09 November 2018 | None | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020200129643
- KR 1020210132988


### Non-patent literature cited in the description


- **REINHOLD SCHWALM.** UV Coatings: Basics, Recent Developments and New Application. Elsevier, 2007, 115
- **ODIAN.** Principle of Polymerization. Wiley, 1981, 203
- **BUCHHOLZ, F.L. ; GRAHAM, A.T.** Modern Superabsorbent Polymer Technology. John Wiley & Sons, 1998, 161